# EUROPÄISCHE PATENTANMELDUNG

(11) **EP 4 681 661 A1**
(43) Veröffentlichungstag der Anmeldung: **21.01.2026**
(21) Anmeldenummer: 25170599.2
(22) Anmeldetag: 15.04.2025
(51) Int. Cl.: A61B 17/16

(54) **BOHRWELLE FÜR EINEN MARKRAUMBOHRER**

(30) Priorität: 18.07.2024 DE 102024120414
(71) Anmelder: Witte, Peter, 24111 Kiel (DE)
(72) Erfinder: Witte, Peter, 24111 Kiel (DE)
(74) Vertreter: Seemann & Partner Patentanwälte mbB

(57) **Zusammenfassung**

Die Erfindung betrifft eine Bohrwelle 10 für einen Markraumbohrer 1, den Markraumbohrer 1, eine impulsverringernde Beschichtung 17, die Verwendung dieser Beschichtung 17 und ein Verfahren zum Wiederaufbereiten des Markraumbohrers 1. Die Bohrwelle 10 umfasst einen längserstreckten Schaft 19, wobei der Schaft 19 an einem ersten Ende 11 einen Bohreranschluss 13 und an einem gegenüberliegenden zweiten Ende 12 einen Antriebsanschluss 14 aufweist. Der Schaft 19 weist einen Wellenkern 18 mit einer durchgehenden, längsaxialen Bohrung 15 auf. Der Wellenkern 18 ist aus Nitinol gefertigt. Eine Mantelfläche 16 des Wellenkerns 18 weist eine impulsverringernde Beschichtung 17 aus Polyetheretherketon auf. Die Beschichtung 17 erstreckt sich von dem ersten Ende 11 bis zum zweiten Ende 12.

## Beschreibung

Die Erfindung betrifft eine Bohrwelle für einen Markraumbohrer. Zudem betrifft die Erfindung einen Markraumbohrer, eine impulsverringernde Beschichtung und die Verwendung einer Beschichtung aus Polyetheretherketon auf einem aus Nitinol gefertigten Schaft einer Bohrwelle eines Markraumbohrers.

Markraumbohrer werden vielfach genutzt, um die Markhöhle im Knochen eines Patienten aufzubohren, damit Implantate eingebracht werden können. Um zu dem aufzubohrenden Bereich zu gelangen, sind die Bohrwellen solcher Markraumbohrer in der Regel flexibel ausgebildet. Die flexiblen Elemente solcher Bohrwellen sind jedoch zumeist anfällig für Keimbelastungen. Aus diesem Grund ist es notwendig, den gesamten Markraumbohrer nach jeder Anwendung gründlich aufzubereiten und zu sterilisieren. Gerade im Bereich der flexiblen Bohrwelle ist die notwendige Aufbereitung und Sterilisierung jedoch nicht immer im erforderlichen Maße möglich, da sich in den Zwischenräumen zwischen den flexiblen Elementen schwer zu entfernende Keime ansetzen können.

Eine Möglichkeit, diesem Problem zu entgehen, besteht darin, den Markraumbohrer als Single Use Bauteil auszubilden und nach jeder Anwendung zu ersetzen. Dies führt jedoch zu hohen Kosten und einem hohen Material- und Fertigungsaufwand.

Die Aufgabe der Erfindung besteht darin, bei der Nutzung eines Markraumbohrers eine gute Aufbereitung und Sterilisierung sicherzustellen, ohne einen zu großen Materialeinsatz und Fertigungsaufwand zu benötigen.

Diese Aufgabe wird gelöst durch eine Bohrwelle für einen Markraumbohrer, umfassend einen längserstreckten Schaft, wobei der Schaft an einem ersten Ende einen Bohreranschluss und an einem gegenüberliegenden zweiten Ende einen Antriebsanschluss aufweist, wobei der Schaft einen Wellenkern mit einer durchgehenden, längsaxialen Bohrung aufweist, wobei der Wellenkern aus Nitinol gefertigt ist, wobei eine Mantelfläche des Wellenkerns eine impulsverringernde Beschichtung aus Polyetheretherketon aufweist, wobei sich die Beschichtung von dem ersten Ende bis zu dem zweiten Ende erstreckt.

Eine Bohrwelle aus Nitinol weist die erforderlichen Materialeigenschaften auf, unter anderem die notwendige Biegsamkeit, damit die Bohrwelle ohne zusätzliche flexible Elemente auskommt. Jedoch hat Nitinol den Nachteil, dass im Falle eines Bruchs der Bohrwelle Splitter entstehen, die in den Körperinnenraum des Patienten gelangen würden und dort großen Schaden anrichten könnten. Um dies zu verhindert, ist die Beschichtung aus Polyetheretherketon vorgesehen. Polyetheretherketon ist auch als PEEK bekannt. Durch eine Beschichtung aus PEEK werden die Splitter des Nitinolschafts im Falle eines Bruchs daran gehindert, in den Körperinnenraum des Patienten einzudringen. Die Beschichtung wirkt somit als impulsverringernde Beschichtung.

Die Bohrwelle ist als Austauschbauteil ausgestaltet. Die Bohrwelle wird somit nach jeder Nutzung ausgewechselt. Um die Kosten und den Materialverbrauch jedoch nicht unnötig hoch zu gestalten, ist lediglich die Bohrwelle selbst als Austauschbauteil ausgebildet. An ihrem ersten Ende weist sie einen Bohreranschluss zum Anschluss eines Bohrkopfes auf und an ihrem zweiten Ende einen Antriebsanschluss zum Anschluss eines Anschlussadapters, mittels dessen die Bohrwelle beispielsweise an eine Bohrmaschine angeschlossen werden kann. Sowohl der Bohrkopf als auch der Anschlussadapter sind so ausgestaltet, dass sie ohne Probleme wieder aufbereitet werden können. Somit können diese Bauteile nach jeder Nutzung wiederverwendet werden. Durch den Bohreranschluss und den Antriebsanschluss der Bohrwelle lassen sich der Bohrkopf und der Anschlussadapter nach der Aufbereitung leicht mit einer neuen Bohrwelle verbinden, um so einen neuen Markraumbohrer zu schaffen, der teils aus wiederaufbereiteten Teilen und teils aus einem Austauschbauteil in Form der Bohrwelle besteht.

Vorzugsweise ist die Mantelfläche durchgehend mit der Beschichtung aus Polyetheretherketon beschichtet. Durch eine durchgehende Beschichtung der gesamten Mantelfläche wird ein Eindringen von Splittern in den Patientenkörper zuverlässig unterbunden. Insbesondere sind auch das erste Ende und das zweite Ende vollständig mit der impulsverringernden Beschichtung aus Polyetheretherketon versehen.

Bevorzugt weist die Beschichtung eine Dicke von wenigstens 0,3 mm, insbesondere wenigstens 0,5 mm, auf. Beispielsweise hat die Beschichtung eine Dicke von etwa 0,5 mm oder etwa 1 mm. Eine solche Schichtdicke ist ausreichend, um ein Eindringen der Nitinolsplitter in den Patientenkörper zu verhindern, ohne dass die Flexibilität der Bohrwelle unnötig eingeschränkt wird.

Gemäß einer Ausführungsform weist der Bohreranschluss einen Mehrkantanschluss für einen Bohrkopf auf. Der Mehrkantanschluss ist beispielsweise als Außensechskantanschluss ausgebildet. Durch einen solchen Anschluss in Form eines Mehrkantanschlusses lässt sich die Bohrwelle zuverlässig und sicher mit dem Bohrerkopf verbinden.

Gemäß einer weiteren Ausführungsform weist der Antriebsanschluss einen Mehrkantanschluss für einen Anschlussadapter auf. Der Mehrkantanschluss ist beispielsweise als Außensechskantanschluss ausgebildet.

Vorzugsweise weist der Wellenkern einen Durchmesser von 6 mm bis 15 mm auf. Der Wellenkern, also der Schaft ohne die Beschichtung, besteht insbesondere vollständig aus Nitinol. Ein solcher Durchmesser des Wellenkerns verschafft der Bohrwelle die notwendige Flexibilität und Stabilität.

Vorzugsweise weist die Bohrung des Schafts einen Durchmesser von 3 mm bis 5,5 mm auf. Durch die Bohrung ist der Schaft kanüliert ausgebildet. Durch die Bohrung wird ein Führungsdraht gezogen, der die Bohrwelle und den Bohrkopf während des Bohrvorgangs sicher führt und dafür sorgt, dass exakt der geplante Bereich der Markhöhle aufgebohrt wird. Die Kanülierung des Markraumbohrers dient somit unter Einsatz des Führungsdrahtes zur richtungsstabilen Führung des Bohrers und zur sicheren Explantation des Markraumbohrers bzw. eines gesamten Markraumbohrersystems.

Gemäß einer Ausführungsform ist der Bohreranschluss und/oder der Antriebsanschluss als verbreiterter Abschnitt ausgebildet, wobei der verbreiterte Abschnitt eine umlaufende, rampenförmige Fläche umfasst. Im Bereich des Bohreranschlusses und/oder des Antriebsanschlusses hat der Schaft somit einen verbreiterten Durchmesser. Zusammen mit der rampenförmigen Fläche dient dies als Haltefläche für einen Anschluss des Bohrkopfes bzw. des Anschlussadapters. Die rampenförmige Fläche hat insbesondere die Form einer Doppelrampe. Insbesondere weist der verbreiterte Abschnitt eine verdrehsichere Kontur auf. Mittels dieser Kontur lässt sich insbesondere der Bohrkopf bzw. der Anschlussadapter an der Bohrwelle einfach montieren.

Die Aufgabe wird zudem gelöst durch einen Markraumbohrer, umfassend eine Bohrwelle nach einer der zuvor diskutierten Ausführungsformen, und einen Bohrkopf, wobei der Bohrkopf einen Schaftanschluss aufweist, der formschlüssig zum Bohreranschluss der Bohrwelle ausgebildet ist. Der Bohrkopf ist insbesondere wiederaufbereitbar. Auf diese Weise wird vorteilhaft ein Markraumbohrer zur Verfügung gestellt, der sowohl eine austauschbare Bohrwelle als auch einen wiederverwendbaren Bohrkopf umfasst. Insbesondere weist der Bohrkopf eine mehrkantförmige Innenbohrung auf, die formschlüssig zum Mehrkantanschluss der Bohrwelle ausgebildet ist. Der Bohrkopf weist insbesondere eine durchgehende, längsaxiale Bohrung auf, die im fixierten Zustand des Bohrkopfs an der Bohrwelle fluchtend mit der längsaxialen Bohrung des Schafts ausgerichtet ist.

Der Markraumbohrer verkörpert die gleichen Vorteile, Merkmale und Eigenschaften wie die zuvor beschriebene Bohrwelle.

Vorzugsweise umfasst der Markraumbohrer einen Anschlussadapter, wobei der Anschlussadapter einen Schaftanschluss aufweist, der formschlüssig zum Antriebsanschluss der Bohrwelle ausgebildet ist. Der Bohrkopf und/oder der Anschlussadapter sind insbesondere lösbar an der Bohrwelle fixierbar. Der Anschlussadapter ist insbesondere wiederaufbereitbar. Somit ist der Anschlussadapter wiederverwendbar. Insbesondere weist der Anschlussadapter eine mehrkantförmige Innenbohrung auf, die formschlüssig zum Mehrkantanschluss der Bohrwelle ausgebildet ist. Der Adapteranschluss weist insbesondere eine durchgehende, längsaxiale Bohrung auf, die im fixierten Zustand des Anschlussadapters an der Bohrwelle fluchtend mit der längsaxialen Bohrung des Schafts ausgerichtet ist.

Gemäß einer Ausführungsform umfasst der Markraumbohrer die Bohrwelle, deren Bohreranschluss und/oder Antriebsanschluss als verbreiterter Abschnitt ausgebildet ist, wobei der verbreiterte Abschnitt eine umlaufende rampenförmige Fläche umfasst, wobei der Markraumbohrer ferner wenigstens einen hülsenförmigen Verriegelungskörper umfasst, wobei der Verriegelungskörper wenigstens ein federndes, nach innen vorstehendes Halteelement aufweist. Die rampenförmige Fläche bzw. Flächen haben insbesondere die Form einer Doppelrampe. Mittels des Verriegelungskörpers lässt sich der Bohrkopf und/oder der Antriebsanschluss einfach und sicher an der Bohrwelle fixieren. Dazu greift das federnde, nach innen vorstehende Halteelement die rampenförmige Fläche, um ein sicheres Anliegen der Bauteile aneinander zu gewährleisten. Das Halteelement ist insbesondere ein Haltekranz Ein derartiger Verriegelungskörper ist beispielsweise aus dem erteilten Patent DE 10 2019 107 198 B4 bekannt. Insbesondere weist der Verriegelungskörper wenigstens eine Verdrehsicherung auf. Die Verdrehsicherung ist insbesondere dazu ausgebildet, mit der Kontur des verbreiterten Abschnitts zusammen kraftschlüssig zu wirken. Bevorzugt weist auch der Bohrkopf eine umlaufende rampenförmige Fläche und insbesondere eine verdrehsichere Kontur auf. Auch der Anschlussadapter weist insbesondere eine umlaufende, rampenförmige Fläche und insbesondere eine verdrehsichere Kontur auf. Die verdrehsicheren Konturen des Bohrkopfs und des Anschlussadapters sind insbesondere jeweils formschlüssig zu der oder den verdrehsicheren Konturen der Bohrwelle ausgebildet. Bevorzugt umfasst der Markraumbohrer zwei Verriegelungskörper, einen zum Anschluss des Bohrkopfs und einen zum Anschluss des Anschlussadapters.

Die Aufgabe wird zudem gelöst durch eine impulsverringernde Beschichtung aus Polyetheretherketon, die auf einen aus Nitinol gefertigten Wellenkern einer Bohrwelle eines Markraumbohrers aufgebracht ist.

Zudem wird die Aufgabe gelöst durch die Verwendung einer Beschichtung aus Polyetheretherketon auf einem aus Nitinol gefertigten Wellenkern einer Bohrwelle eines Markraumbohrers, um das Eindringen von Nitinolsplittern in den Patientenkörper zu verhindern.

Die impulsverringernde Beschichtung und die Verwendung dieser Beschichtung verkörpern die gleichen Vorteile, Merkmale und Eigenschaften wie die zuvor beschriebene Bohrwelle und der zuvor beschriebene Markraumbohrer.

Schließlich wird die Aufgabe gelöst durch Verfahren zum Wiederaufbereiten eines Markraumbohrers, wobei ein Bohrkopf und ein Anschlussadapter wiederaufbereitet und wiederverwendet werden, wobei eine alte Bohrwelle, die als einmalbenutzbares Austauschteil ausgebildet ist, ausgetauscht und durch eine neue Bohrwelle ersetzt wird, wobei der wiederaufbereitete Bohrkopf und der wiederaufbereitete Anschlussadapter mit der neuen Bohrwelle zu einem wiederaufbereiteten Markraumbohrer kombiniert werden.

Die Verfahren zum Wiederaufbereiten eines Markraumbohrers verkörpern die gleichen Vorteile, Merkmale und Eigenschaften wie die zuvor beschriebene Bohrwelle, der zuvor beschriebene Markraumbohrer die zuvor beschriebene impulsverringernde Beschichtung und die zuvor beschriebene Verwendung der Beschichtung.

Gemäß einer Ausführungsform handelt es sich bei der alten Bohrwelle und der neuen Bohrwelle jeweils um eine Bohrwelle nach einer der zuvor beschriebenen Ausführungsformen und bei dem Markraumbohrer um einen Markraumbohrer nach einer der zuvor beschriebenen Ausführungsformen.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte perspektivische Explosionsdarstellung eines Markraumbohrers mit wiederaufbereitbarem Bohrkopf, wiederaufbereitbarem Anschlussadapter und austauschbarer Bohrwelle,
- Fig. 2: eine schematisch vereinfachte Ansicht des Markraumbohrers aus Fig. 1 im zusammengesetzten Zustand,
- Fig. 3: eine schematisch vereinfachte Seitenansicht des Markraumbohrers aus den Fig. 1 und 2,
- Fig. 4: eine schematisch vereinfachte Querschnittsansicht in der Richtung A:A und
- Fig. 5: die Querschnittsansicht aus Fig. 4 ohne den Verriegelungskörper.

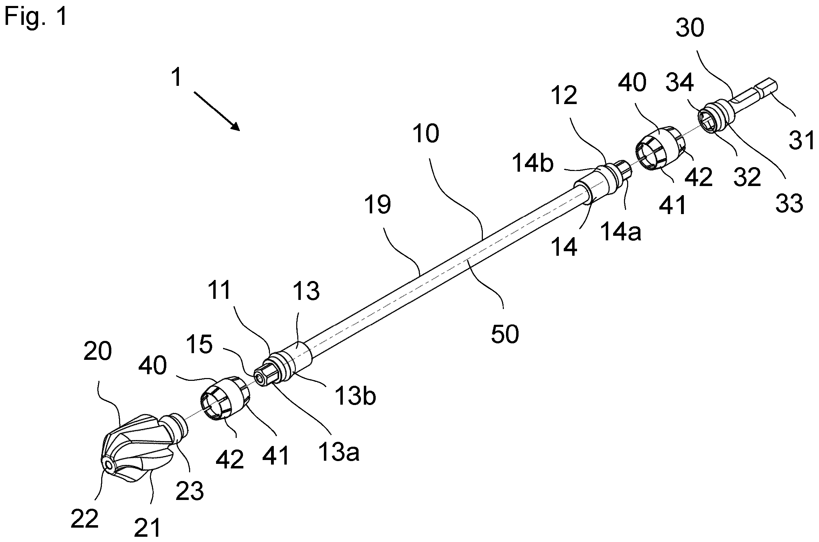

Fig. 1 zeigt schematisch vereinfacht einen Markraumbohrer 1 in einer perspektivischen Explosionsansicht. Der Markraumbohrer 1 umfasst eine Bohrwelle 10, einen Bohrkopf 20, einen Anschlussadapter 30 und zwei Verrieglungskörper 40. Der Bohrkopf 20 weist eine Reihe Schneiden 21 auf, um die Markhöhle im Knochen eines Patienten aufzubohren. Zu diesem Zweck umfasst der Bohrkopf 20 auch eine längsaxiale Bohrung 22, die sich vollständig durch den Bohrkopf 20 erstreckt. Die Bohrung 22 dient zur Aufnahme eines nicht dargestellten Führungsdrahts, der sicherstellt, dass der gesamte Markraumbohrer 1 nach Beendigung des Bohrvorgangs sicher entfernt werden kann. Zudem dient die Bohrung 22 zur richtungsstabilen Führung des Bohrkopfs 20 während des Bohrvorgangs. Hinter den Schneiden 21 weist der Bohrkopf 20 einen hohlzylinderförmigen Abschnitt auf, auf dem eine umlaufende rampenförmige Fläche 23 ausgebildet ist, die zur Fixierung des Bohrkopfs 20 an der Bohrwelle 10 vorgesehen ist. Die rampenförmige Fläche 23 hat in dieser Ausführungsform die Form einer Doppelrampe. Dieser Abschnitt ist somit als Schaftanschluss für die Bohrwelle 10 ausgebildet.

Die Bohrwelle 10 ist flexibel ausgebildet und dazu ausgestaltet, die Drehmomente aufzunehmen, die am Markraumbohrer 1 anliegen. Dazu ist ein Schaft 19 der Bohrwelle 10 aus Nitinol gefertigt, welches der Bohrwelle 10 die nötige Flexibilität und Stabilität verleiht. Um im Falle eines Bruchs des Schafts 19 ein Eindringen von Nitinolsplittern in den Patientenkörper zu verhindern, ist eine Beschichtung auf den Schaft 19 aufgebracht, die aus Polyetheretherketon (PEEK) besteht. Zum Anschluss des Bohrkopfs 20 und des Anschlussadapters 30 umfasst die Bohrwelle 10 an einem ersten Ende 11 einen Bohreranschluss 13 und einem zweiten Ende 12 einen Antriebsanschluss 14. Der Bohreranschluss 13 weist eine verdrehsichere Kontur in Form eines Mehrkantanschlusses 13a auf, der in eine aus perspektivischen Gründen nicht sichtbare verdrehsichere Kontur in Form einer Mehrkantaufnahme im Bohrkopf 20 greift. Der Bohreranschluss 13 ist zudem als verbreiterter Abschnitt mit höherem Durchmesser als der Rest des Schafts 19 ausgebildet und weist eine umlaufende, doppelrampenförmige Fläche 13b auf. Zur Fixierung des Bohrkopfs 20 an der Bohrwelle 10 ist ein Verriegelungskörper 40 vorgesehen, der hülsenförmig ausgebildet ist und an beiden Enden jeweils ein federndes, nach innen vorstehendes Halteelement 41, 42 aufweist. Bei diesem Halteelement 41, 42 handelt es sich beispielsweise um einen Haltekranz. Das Halteelement 41, 42 greift hinter die rampenförmigen Flächen 13b, 23 und hält so den Bohrkopf 20 an der Bohrwelle 10.

Der Antriebsanschluss 14 ist analog zum Bohreranschluss 13 ausgebildet und weist eine verdrehsichere Kontur in Form eines Mehrkantanschluss 14a und eine doppelrampenförmige Fläche 14b auf. Ebenso weist der Anschlussadapter 30 eine verdrehsichere Kontur in Form einer mehrkantförmigen Innenbohrung 34 auf, die formschlüssig zum Mehrkantanschluss 14a der Bohrwelle 10 ausgebildet ist. Die Außenfläche des Anschlussadapters 30 umfasst zudem eine doppelrampenförmige Fläche 33, die wie zuvor beschrieben einen Schaftanschluss bildet und mit einem Halteelement 42 eines weiteren Verriegelungskörpers 40 wechselwirkt, um den Anschlussadapter 30 an der Bohrwelle 10 zu fixieren.

Genau wie der Bohrkopf 20 weist auch die Bohrwelle 10 eine längsaxiale Bohrung 15 und der Anschlussadapter 30 eine längsaxiale Bohrung 32 auf, die sich jeweils vollständig in Richtung einer Längsachse 50 durch die Bauteile erstrecken. Der Markraumbohrer 1 ist somit kanüliert. Auf diese Weise kann der Führungsdraht durch alle Bauteile 10, 20, 30 durchgezogen werden, um diese zu führen und zu entnehmen. Zur Aufnahme des Drehmoments von einer nicht gezeigten Antriebsmaschine bzw. Bohrmaschine ist das hintere Ende des Anschlussadapters 30 als Maschinenanschluss 31 ausgeformt.

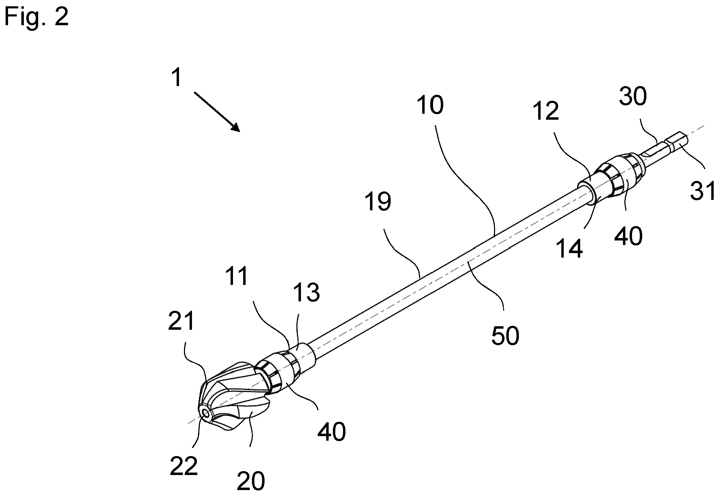

Fig. 2 zeigt den Markraumbohrer 1 im zusammengesetzten Zustand. In diesem Zustand sind der Bohrkopf 20 und der Anschlussadapter 30 mittels der Verriegelungskörper 40 an der Bohrwelle 10 fixiert. Auf diese Weise umfasst der resultierende Markraumbohrer 1 mehrere wiederaufbereitbare Einzelteile, nämlich den Bohrkopf 20, den Anschlussadapter 30 und den Verriegelungskörper 40. Andererseits umfasst der Markraumbohrer 1 die einmal benutzbare Bohrwelle 10. Auf diese Weise kann auf eine schwierige und aufwendige Aufbereitung der Bohrwelle 10 verzichtet werden, ohne den gesamten Markraumbohrer 1 austauschen zu müssen. Insbesondere der aufwendig herzustellende Bohrkopf 20 kann auf diese Weise wiederverwendet werden, ohne dass die Gefahr besteht, dass sich Keime in schwer zu erreichenden Stellen der Bohrwelle 10 auch trotz einer Aufbereitung festsetzen.

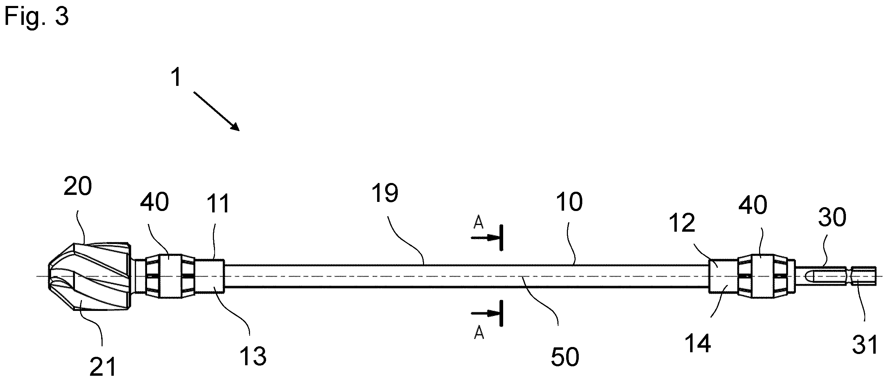

Fig. 3 zeigt den Markraumbohrer 1 aus den Fig. 1 und 2 in einer Seitenansicht. In dieser Ansicht wird deutlich, wie der Bohrkopf 20 und der Anschlussadapter 30 mittels der Verriegelungskörper 40 an dem Bohreranschluss 13 bzw. dem Antriebsanschluss 14 des Schafts 19 anliegen. Zudem ist eine Schnittlinie A:A dargelegt, auf die im Folgenden eingegangen wird.

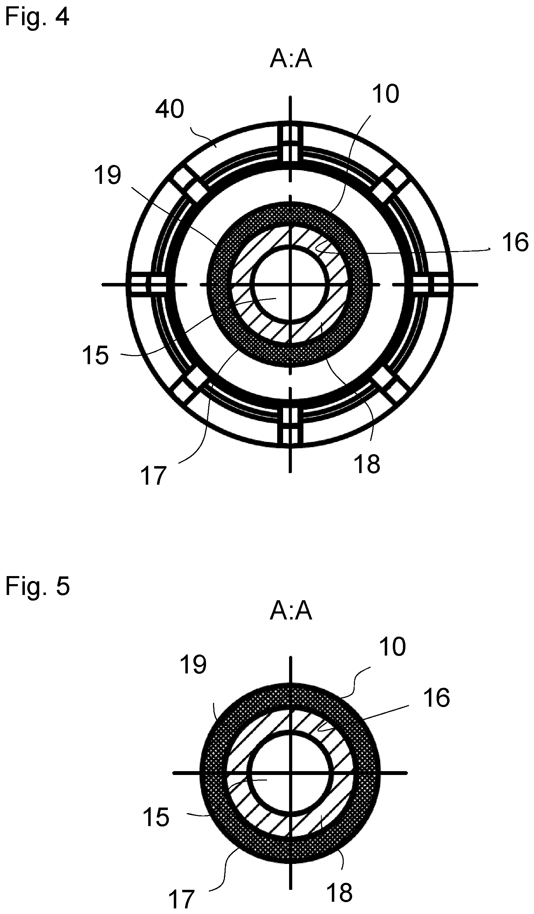

Fig. 4 zeigt eine Querschnittsansicht entlang der Schnittsebene A:A. Da die Blickrichtung in dieser Darstellung in Richtung des Anschlussadapters 30 gerichtet ist, ist in Fig. 4 ebenfalls der Verriegelungskörper sichtbar, obwohl dieser weit hinter der eigentlichen Querschnittsebene A:A liegt. Fig. 5 zeigt daher die Ansicht aus Fig. 4 ohne den Verriegelungskörper 40. In beiden Ansichten ist zu erkennen, dass der Schaft 19 der Bohrwelle 10 einen Wellenkern 18 aus Nitinol umfasst, in dem zentral eine Bohrung 15 eingebracht ist. Der Wellenkern 18 ist entlang seiner Mantelfläche 16 mit einer impulsverringernden Beschichtung 17 aus Polyetheretherketon (PEEK) beschichtet. Wie zuvor dargelegt, verhindert diese Beschichtung 17, dass bei einem Bruch des Schafts 19 Nitinolsplitter des Wellenkerns 18 nach außen gelangen.

In den Zeichnungen sind jeweils gleiche oder gleichartige Elemente und/oder Teile mit denselben Bezugsziffern versehen, so dass von einer erneuten Vorstellung jeweils abgesehen wird.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 1: Markraumbohrer
- 10: Bohrwelle
- 11: erstes Ende
- 12: zweites Ende
- 13: Bohreranschluss
- 13a: Mehrkantanschluss
- 13b: rampenförmige Fläche
- 14: Antriebsanschluss
- 14a: Mehrkantanschluss
- 14b: rampenförmige Fläche
- 15: Bohrung
- 16: Mantelfläche
- 17: Beschichtung
- 18: Wellenkern
- 19: Schaft
- 20: Bohrkopf
- 21: Schneide
- 22: Bohrung
- 23: rampenförmige Fläche
- 30: Anschlussadapter
- 31: Maschinenanschluss
- 32: Bohrung
- 33: rampenförmige Fläche
- 34: mehrkantförmige Innenbohrung
- 40: Verriegelungskörper
- 41: Halteelement
- 42: Halteelement
- 50: Längsachse

## Patentansprüche

1. Bohrwelle (10) für einen Markraumbohrer (1), umfassend einen längserstreckten Schaft (19), wobei der Schaft (19) an einem ersten Ende (11) einen Bohreranschluss (13) und an einem gegenüberliegenden zweiten Ende (12) einen Antriebsanschluss (14) aufweist, wobei der Schaft (19) einen Wellenkern (18) mit einer durchgehenden, längsaxialen Bohrung (15) aufweist, wobei der Wellenkern (18) aus Nitinol gefertigt ist, wobei eine Mantelfläche (16) des Wellenkerns (18) eine impulsverringernde Beschichtung (17) aus Polyetheretherketon aufweist, wobei sich die Beschichtung (17) von dem ersten Ende (11) bis zu dem zweiten Ende (12) erstreckt.

2. Bohrwelle (10) nach Anspruch 1, **dadurch gekennzeichnet, dass** die Mantelfläche (16) durchgehend mit der Beschichtung (17) aus Polyetheretherketon beschichtet ist.

3. Bohrwelle (10) nach Anspruch 1 oder 2, **dadurch gekennzeichnet, dass** die Beschichtung (17) eine Dicke von wenigstens 0,3 mm, insbesondere wenigstens 0,5 mm, aufweist.

4. Bohrwelle (10) nach einem der Ansprüche 1 bis 3, **dadurch gekennzeichnet, dass** der Bohreranschluss (13) einen Mehrkantanschluss für einen Bohrkopf (20) aufweist.

5. Bohrwelle (10) nach einem der Ansprüche 1 bis 4, **dadurch gekennzeichnet, dass** der Antriebsanschluss (14) einen Mehrkantanschluss für einen Anschlussadapter (30) aufweist.

6. Bohrwelle (10) nach einem der Ansprüche 1 bis 5, **dadurch gekennzeichnet, dass** der Wellenkern (18) einen Durchmesser von 6 mm bis 15 mm aufweist.

7. Bohrwelle (10) nach einem der Ansprüche 1 bis 6, **dadurch gekennzeichnet, dass** die Bohrung (15) des Schafts (19) einen Durchmesser von 3 mm bis 5,5 mm aufweist.

8. Bohrwelle (10) nach einem der Ansprüche 1 bis 7, **dadurch gekennzeichnet, dass** der Bohreranschluss (13) und/oder der Antriebsanschluss (14) als verbreiterter Abschnitt ausgebildet ist, wobei der verbreiterte Abschnitt eine umlaufende, rampenförmige Fläche (13b, 14b) umfasst.

9. Markraumbohrer (1), umfassend eine Bohrwelle (10) nach einem der Ansprüche 1 bis 8 und einen Bohrkopf (20), wobei der Bohrkopf (20) einen Schaftanschluss aufweist, der formschlüssig zum Bohreranschluss (13) der Bohrwelle (10) ausgebildet ist.

10. Markraumbohrer (1) nach Anspruch 9 umfassend einen Anschlussadapter (30), wobei der Anschlussadapter (30) einen Schaftanschluss aufweist, der formschlüssig zum Antriebsanschluss (14) der Bohrwelle (10) ausgebildet ist.

11. Markraumbohrer (1) nach Anspruch 9 oder 10, umfassend die Bohrwelle (10) aus Anspruch 8 und wenigstens einen hülsenförmigen Verriegelungskörper (40), wobei der Verriegelungskörper (40) wenigstens ein federndes, nach innen vorstehendes Halteelement (41, 42) aufweist.

12. Impulsverringernde Beschichtung (17) aus Polyetheretherketon, die auf einen aus Nitinol gefertigten Wellenkern (18) einer Bohrwelle (10) eines Markraumbohrers (1) aufgebracht ist.

13. Verwendung einer Beschichtung (17) aus Polyetheretherketon auf einem aus Nitinol gefertigten Wellenkern (18) einer Bohrwelle (10) eines Markraumbohrers (1), um das Eindringen von Nitinolsplittern in den Patientenkörper zu verhindern.

14. Verfahren zum Wiederaufbereiten eines Markraumbohrers (1), wobei ein Bohrkopf (20) und ein Anschlussadapter (30) wiederaufbereitet und wiederverwendet werden, wobei eine alte Bohrwelle (10), die als einmalbenutzbares Austauschteil ausgebildet ist, ausgetauscht und durch eine neue Bohrwelle (10) ersetzt wird, wobei der wiederaufbereitete Bohrkopf (20) und der wiederaufbereitete Anschlussadapter (30) mit der neuen Bohrwelle (10) zu einem wiederaufbereiteten Markraumbohrer (1) kombiniert werden.

15. Verfahren nach Anspruch 14, **dadurch gekennzeichnet, dass** es sich bei der alten Bohrwelle (10) und der neuen Bohrwelle (10) jeweils um eine Bohrwelle (10) nach einem der Ansprüche 1 bis 8 und bei dem Markraumbohrer (1) um einen Markraumbohrer (1) nach einem der Ansprüche 9 bis 11 handelt.
